# EUROPEAN PATENT SPECIFICATION

(11) **EP 2 679 266 B1**
(45) Date of publication and mention of the grant of the patent: **08.06.2016**
(21) Application number: 12174257.1
(22) Date of filing: 29.06.2012
(51) Int. Cl.: A61M 16/00, A61M 16/04, A61M 16/06, A61M 16/08

(54) **Coupling system for securing a nasal cushion to a respiratory mask**
Kopplungssystem zur Sicherung eines Nasenkissens an einer Atemmaske
Système de couplage destiné à fixer un coussinet nasal à un masque respiratoire

(43) Date of publication of application: 01.01.2014
(73) Proprietor: Air Liquide Medical Systems, 92160 Antony (FR)
(72) Inventor: Alberici, Luca, 25128 Brescia (IT); Masserdotti, Fulvio, 25075 Brescia (IT); Sandoni, Giuseppe, 25124 Brescia (IT)
(74) Representative: Pittis, Olivier

(56) References cited:
- EP-A2- 2 022 528
- DE-U1-202004 021 758
- US-A1- 2008 053 450
- US-A1- 2011 162 655
- US-A1- 2012 132 209

## Description

The invention concerns a nasal cushion for a respiratory mask comprising coupling means that allows a quick and easy connecting of the nasal cushion to the mask body, and a respiratory mask equipped with such a nasal cushion.

Nasal pillow masks are used for non invasive ventilation for treating or preventing acute respiratory failures or disorders, as can occur, for instance, for sleep apnoea disorders or the like.

Such a mask generally comprises a rigid or semi-rigid hollow shell or mask body defining an internal breathing chamber or volume, wherein a respiratory gas, such as air under pressure, is introduced via an inlet port through a short tube assembly to which is connected a gas feeding line by means of a tubular connector.

The rigid or semi-rigid mask body is fixed to a headgear for correctly positioning, maintaining and/or securing the mask on the head of a patient.

The mask body further comprises a peripheral border which is designed to couple with a nasal pillow cushion or nasal cushion by means of coupling means.

Nasal cushions are commonly made of soft material, such as silicone and comprise two prongs or cannulae that are configured to engage with the internal contour of the nostrils when being partially inserted into the nostrils of the patient.

Examples of nasal pillow masks equipped with nasal cushions are given by documents EP-A-2140902, EP-A-1603619, EP-A-2051761, EP-A-2259827, EP-A-2349428 and WO-A-2009/151344.

As nasal pillow masks are used for the treatment or prevention of acute respiratory failures, they are usually worn overnight and even during the day by the patients, and quality of treatment and comfort of use are hence very important features.

One problem with such masks concerns the gas leaks that may appear between the nasal cushion and the rigid mask body, when the coupling of the cushion to the mask is not strong enough and not sufficiently tight.

Indeed, with many existing masks, the connection between the cushion and the mask body is poor so that leaks often appear during the use of the mask, creating parasitic gas flows and a discomfort for the patient.

Further, in some cases, the poor connection or coupling of the cushion to the mask body leads to unwanted disconnection or release of the cushion from the rigid mask body, that obligates the patient to reconnect both parts. This can be very annoying for the patient, especially during the night.

EP-A-2022528 discloses a nasal cushion with a pair of nasal prongs that can be connected to a respiratory mask (see Fig. 16-15-10). The coupling is obtained by means of an outer wall and an inner wall separated from each other by an intermediary spacing, wherein the mask border is introduced and squeezed between said inner and outer walls. US-A-2012/0132209 teaches a similar nasal cushion structure (see Fig. 17d).

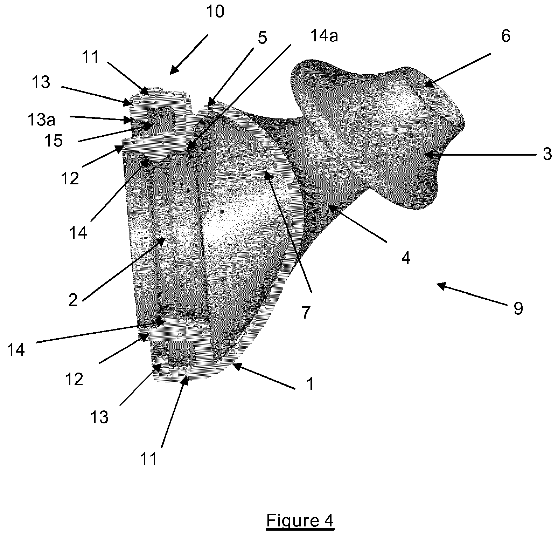

Further, US-A-2008/0053450 discloses a respiratory mask comprising a nasal cushion shaped for recovering the entire of the nose of the patient. The cushion is fixed to the mask body by a particular coupling structure (see Fig. 4B) comprising an outer wall and an inner wall separated from each other by an intermediary spacing, wherein the cushion border is introduced and squeezed. The outer wall comprises an expansion projecting toward the inner wall and forming a hook-like part. Here, the coupling structure is however carried by the mask body itself.

Hence, the main goal of the present invention is to improve the connection or coupling of the nasal cushion to the mask body for avoiding or minimizing the appearance of leaks and the undesired disconnecting of the nasal cushion from the mask body.

The solution of the present invention is defined in claim 1.

Depending on the considered embodiment, the nasal cushion of the present invention can comprise one or more of the following features :
- the outer wall and inner wall are parallel or quasi-parallel to each other.
- the outer wall is facing the inner wall.
- the second radial expansion forms a boss on the inner surface of the inner wall.
- the outer wall and the inner wall are formed at the end of the peripheral border.
- the outer wall and the inner wall are formed at the end of the peripheral border over the entire periphery of said peripheral border.
- the first radial expansion of the outer wall comprises a bevelled front face.
- the hollow body, the pair of nasal prongs, the peripheral border, the outer wall and the inner wall are formed in one-piece, preferably moulded in one-piece.
- each nasal prong comprises an internal gas passage in fluid communication with the internal chamber of the hollow body, and further comprises an outlet orifice fluidly communicating with the internal gas passage.
- each nasal prongs comprises a flexible bellow portion allowing each prong to pivot so as to be orientated with respect to the hollow body.
- the flexible bellow portions are located at the proximal end of the nasal prongs.
- each nasal prong comprises an enlarged head, at its distal end, comprising the outlet orifice.
- said enlarged heads have a generally tronconical or quasi-tronconical shape.
- the hollow body and the prongs are made of a soft deformable material, preferably of silicone.
- the hollow body, the pair of nasal prongs, the peripheral border, the outer wall and the inner wall are made of a resilient flexible material, such as silicone or an elastomeric material.
- each flexible bellow portion comprises at least one recess, preferably said at least one recess has an annular shape.
- said at least one recess corresponds to a radial projection of the peripheral wall of each nasal prong into the internal gas passage of each nasal prong.
- the enlarged heads are made of a soft deformable material, preferably of silicone, for ensuring a good gas tightness when the heads of the prongs are inserted into the nostrils of the user, i.e., of the patient.
- the hollow body and the pair of nasal prongs are made of silicone.
- each bellow portion comprises one recess forming a groove extending over the entire circumference of each prong.
- each bellow portion comprises several recesses, preferably the recesses are arranged so as to be in successive parallel planes.

The present invention also concerns a nasal respiratory mask comprising a mask body and a nasal cushion according to the present invention, and further comprising a headgear with straps or similar.

Advantageously, the mask body comprises an abutment structure cooperating with the first radial expansion (hook-like structure) of the cushion and a lodging having a wall portion cooperating with the second radial expansion of the cushion, when the nasal cushion is coupled to the mask body.

The present invention will be better understood thanks to the following description made in reference to the accompanying drawings among which :
- Figure 1 shows an embodiment of a nasal mask equipped with a nasal cushion according to the present invention,
- Figure 2 represents the body and part of the headgear of the nasal mask of Figure 1,
- Figure 3 represents an embodiment of a nasal cushion according to the present invention that equips the mask of Figure 1,
- Figure 4 is a view in cross section of the nasal cushion of Figure 3,
- Figure 5 illustrates the coupling of the nasal cushion of Figure 3 to a nasal mask,
- Figure 6 is an enlarged view of the coupling structure of the nasal cushion of Figure 5,
- Figure 7 shows the mechanical interactions between the mask and the cushion after their coupling and
- Figure 8 is a side view of Figure 2.

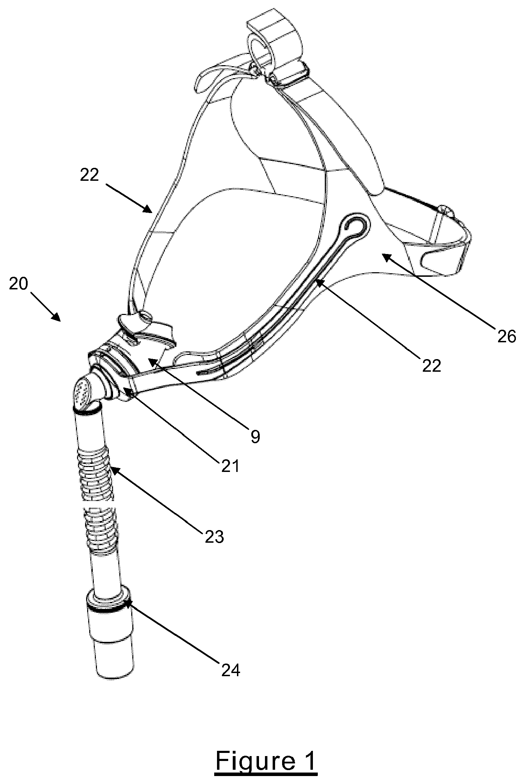

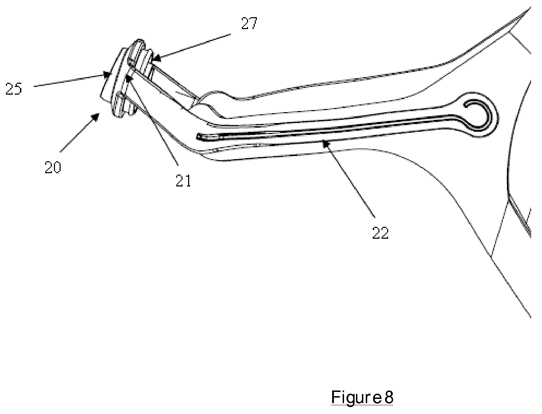

As shown in Figures 1, 2 and 8, the nasal respiratory mask 20 according to the present invention comprises a rigid or semi-rigid hollow shell or mask body 21, for instance made of a polymeric material, defining an internal breathing chamber or volume, wherein a respiratory gas, such as air under pressure, is introduced via an inlet port 25, by means of a flexible tube assembly 23 that is connected to a gas feeding line (non shown) by means of a tubular connector 24 or similar.

The gas inlet orifice 25 is arranged at the centre of the mask body 21 and through its wall thereby allowing air under pressure or any other gas to be introduced in the breathing chamber of the mask body 21.

The rigid or semi-rigid mask body 21 further comprises two lateral arms 22, i.e. one left arm and one right arm, which extend away form the mask body 21, and are fixed to a headgear 26 comprising flexible straps, typically made of fabric, and connecting elements, such as buckles or similar.

The mask body 1 is preferably made of a polymeric material, such as polycarbonate (PC), polypropylene (PP), ABS, nylon or polystyrene (PS).

The mask body 21 further comprises a peripheral border 27 which is designed and/or configured to couple with coupling means, i.e. a connecting structure 11, 13; 12, 14, arranged on the nasal cushion 9 so that the nasal 9 cushion can be made integral with the mask body 21 of the mask 20, i.e. firmly attached to the mask body 21, and so that no gas leaks appear in-between.

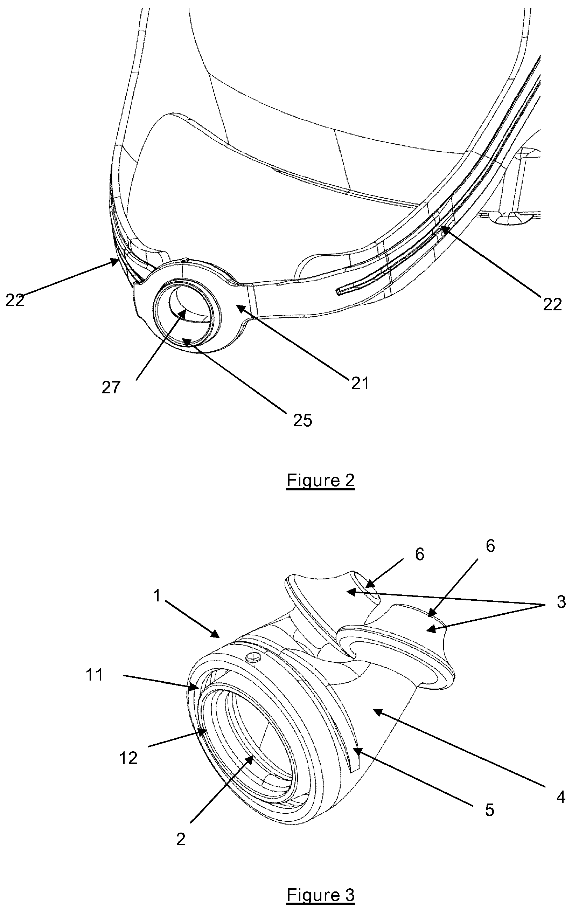

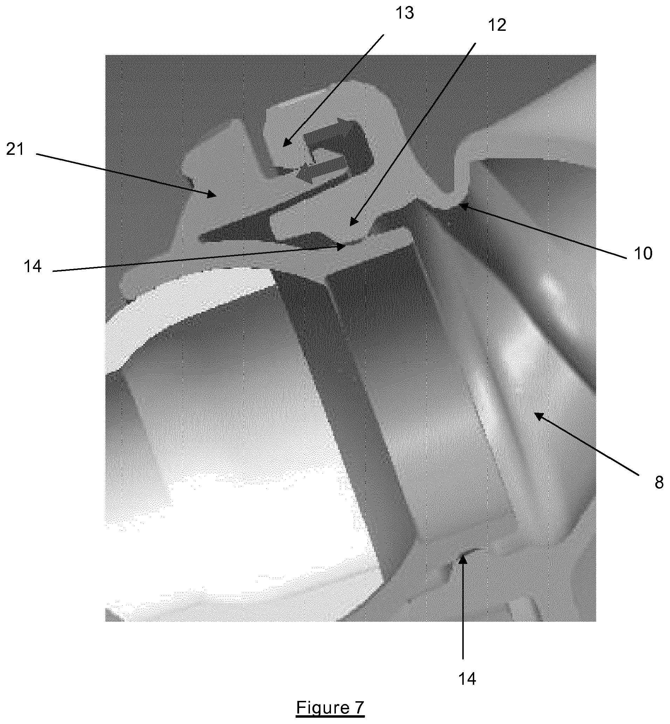

Actually, the nasal cushion 9 according to the present invention, as shown in Figures 3 to 8, comprises a hollow body 1 with an internal chamber 2, and a pair of nasal prongs 4 each connected to and integral with the hollow body 1.

The hollow body 1 of the nasal cushion 9 further comprises a peripheral border 10 configured so as to form a tri-dimensional connecting structure 11, 13, 12, 14 that is used for fixing/coupling the hollow body 1 to the mask body 21 so that the hollow body 1 of the nasal cushion 9 is firmly attached to the mask body 21.

More precisely, the connecting structure 11, 13, 12, 14 comprises an outer wall 11 and an inner wall 12 separated from each other by an intermediary spacing 15, i.e., an empty space. For instance, the end of the hollow body 1 can be divided in 2 sub-walls forming said inner and outer walls 11, 12.

The outer wall 11 carries a first radial expansion 13 projecting in the intermediary spacing 15 toward the inner wall 12. That is to say that the first radial expansion 13 constitutes a small part that projects away from the inner surface of the outer wall 11 facing the intermediary spacing 15, and in the direction of the inner wall 12 as clearly shown in Figure 4.

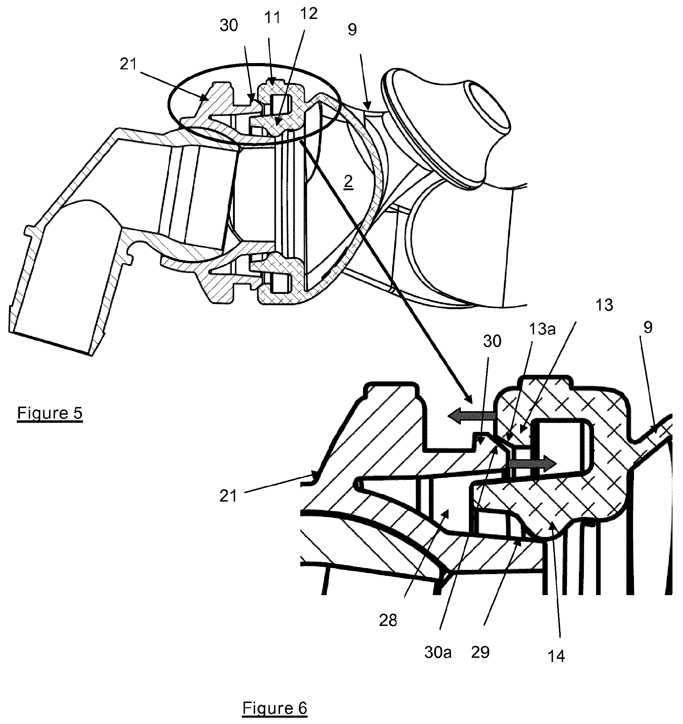

Said first radial expansion 13 constitutes a hook-like structure which comprises a bevelled front face 13a that cooperates with an abutment structure 30 carried by the mask body 21 as shown in Figures 5 and 6, when the cushion 9 is coupled to the mask body 21 as explained below.

Further, the inner wall 12 comprises a second radial expansion 14 that projects in the internal chamber 2 of the hollow body 1, i.e. toward the interior of the nasal cushion 9. Said second radial expansion 14 forms a boss structure on the inner surface 14a of the inner wall 12 that cooperates with a wall portion of the mask body 21, when the cushion 9 is set in place into the mask body 21.

Actually, the external and inner walls 11, 12 as well as the first and second radial expansions 13, 14 form together a tri-dimensional coupling structure that is able to cooperate with the mask body 21, especially with the peripheral border 27 of the mask body 21, for ensuring a good and firm connection or coupling of the nasal cushion 9 to the mask body 21, thereby obtaining also an efficient gas tightness.

In this aim, the peripheral connecting border 27 of the mask body 21 of the mask 20 is configured so as to match the peripheral border 10 of the nasal cushion 9. In other words, their respective profiles or shapes are complementary so as to cooperate together and provide an efficient and firm coupling between the mask body 21 and the nasal cushion 9. The specific structure of the peripheral connecting border 27 of the hollow body 21 of the mask 20 is detailed below.

Generally speaking, each nasal prong 4 is a kind of nasal cannula or small tube comprising an internal gas passage 7 in fluid communication with the internal chamber of the hollow body 21 of the mask, as shown in Figures 3 and 4, so as to be able to convey and distribute gas under pressure to the patient's nostrils, while the prongs 4 are inserted into the patient's nostrils. The gas is delivered by each prong 3 by an outlet orifice 6 arranged at its distal end, i.e. on each prong head 3, which is in fluid communication with the internal gas passage of the prong 12.

Further, for ensuring an efficient and auto-positioning of the nasal mask into the patient's nostrils, especially a correct positioning and orientation of the pair of prongs 4, that avoids gas leaks and ensures a good gas distribution and, consequently, an efficient therapy, each nasal prong 4 comprises a flexible bellow portion 5 allowing each prong 4 to pivot with respect to the hollow body 1 of the cushion 9. The flexible bellow portions 5 are located at the proximal end of the nasal prongs 4, i.e. in the vicinity of the hollow body 1 of the cushion 9.

Thanks to the flexible bellow portion 5, the nasal prongs 4 are pivotable and flexible with respect to the hollow body 1 of the cushion 9. This allows the nasal prongs 4 to efficiently pivot, i.e. to be mobile so that they can bend, bow, oscillate or similar, while being maintained by their proximal end, so as to modify their orientations with respect to the cushion hollow body 1.

The nasal prongs 4, including their flexible bellow portion 5, are preferably made of a flexible material, such as thermoplastic rubber or elastomeric polymer. Preferably, they are made of silicone or the like. Preferably, the nasal prongs 4, their head 3 and the cushion body 1 are be moulded in one piece, preferably they are made of silicone or the like.

The flexible bellow portion 5 of each prong 4 comprises one (or several) recess having preferably an annular or semi-annular shape. In other words, the recess forms a groove in the wall of each prong 4, preferably extending over the entire external circumference of each prong 4. Actually, each recess corresponds to a radial projection of the peripheral wall of said nasal prongs 4 into the internal gas passages 7 of said nasal prongs.

Further, each nasal prong 4 of the nasal pillow cushion 9 comprises, at its distal end, an enlarged head 3 configured to have a generally tronconical or quasi-tronconical shape as shown in Figures 3-5.

Preferably, said enlarged heads 3 have an elliptical surface and are made of a thin resilient material, such as a thin silicone wall, so that the enlarged heads inflate like a balloon and are deformed by the gas pressure exerted on their inner wall, when a pressurized gas passes through them, thereby creating an efficient seal between the surface of each enlarged head 3 and the inner wall of the nostrils of the patient. Indeed, when the enlarged heads 3 are inflated by gas, they match the inner contour of the patient's nostrils, thus creating a seal.

Furthermore, as detailed in Figures 3-7, the outer wall 11 and the inner wall 12 forming the coupling structure of the cushion 9 are facing each other and are further substantially parallel or quasi-parallel to each other.

The outer wall 11 and the first radial expansion 13 form a hook-like structure that is able to cooperate with an abutment structure 30 arranged on the mask body 21 so as to maintain the nasal cushion 9 firmly attached to the mask body 21.

The firm coupling is further improved thanks to the second radial expansion 14 of the inner wall 12 that forms a projecting boss that cooperates with a wall portion 29 of a lodging 28 of the mask body 21, when the inner wall 12 is inserted into the lodging 28, as shown in Figures 5, 6 and 7.

Actually, thanks to the intrinsic properties of the silicone rubber constituting the nasal cushion 9, including the projecting boss forming the second radial expansion 14, the nasal cushion 9 does not slide on the surface of the wall portion 29, when the inner wall 12 is inserted into the lodging 28.

In other words, the nasal cushion 9 can be firmly maintained on the mask body 21 thanks to the combination of actions of the hook-like structure 13 of the outer wall 11 of the cushion 9 that cooperates with the abutment structure 30 of the mask body 21, and of the projecting boss forming the second radial expansion 14 of the cushion 9 that comes into contact, in a non-sliding way, with the surface of the wall portion 29 of the mask body 21, while the cushion 9 is coupled to the mask body 21.

The boss 14 acts also as a gasket for ensuring the gas tightness of the mask.

Further, for ensuring an easy coupling or securing of the cushion 9 to the mask body 21, the first radial expansion 13 of the outer wall 11 comprises a first bevelled front face 13a that can slide against a corresponding bevelled front face 30a of the abutment 30 as shown in Figure 6.

The outer wall 11 and an inner wall 12 extend over the entire periphery of the nasal cushion 9 as shown in Figure 4.

The nasal mask and cushion according to the present invention are useable for treating respiratory failures or disorders, such as sleep apnoea or the like.

## Claims

1. Respiratory mask (20) comprising a mask body (21) fixed to a nasal cushion (9), said nasal cushion (9) comprising a hollow body (1) with an internal chamber (2), and a pair of nasal prongs (4) each connected to and integral with the hollow body (1), said hollow body (1) further comprising a peripheral border (10) comprising a connecting structure (11, 13; 12, 14) for fixing or coupling the hollow body (1) to the mask body (20), the connecting structure (11, 13; 12, 14) comprising an outer wall (11) and an inner wall (12) separated from each other by an intermediary spacing (15),
**characterized in that**:
- the outer wall (11) of the nasal cushion (9) comprises a first radial expansion (13) projecting in the intermediary spacing (15) toward the inner wall (12), and said inner wall (12) comprises a second radial expansion (14) projecting in the internal chamber (2) of the hollow body (1),
- the outer wall (11) and the first radial expansion (13) of the nasal cushion (9) form a hook-like structure, said first radial expansion (13) comprising a first bevelled front face (13a),
- the second radial expansion (14) of the nasal cushion (9) forms a boss on the inner surface (14a) of the inner wall (12),
- and the mask body (21) comprises :
. an abutment structure (30) comprising a second bevelled front face (30a), and cooperating with the first radial expansion (13) of the cushion (9), when the cushion (9) is set in place into the mask body (21), and
. a lodging (28) having a wall portion (29) cooperating with the boss structure on the inner surface (14a) of the inner wall (12) of the second radial expansion (14) of the cushion (9), when the cushion (9) is set in place into the mask body (21),
thereby firmly maintaining the nasal cushion (9) on the mask body (21) thanks to the combination of actions of the hook-like structure (13) of the outer wall (11) of the cushion (9) that cooperates with the abutment structure (30) of the mask body (21), and of the projecting boss of the second radial expansion (14) of the cushion (9) that comes into contact, in a non-sliding way, with the surface of the wall portion (29) of the mask body (21), while the cushion (9) is coupled to the mask body (21),
said boss further acting as a gasket ensuring a gas tightness between the second radial expansion (14) of the cushion (9) and the wall portion (29) of the mask body (21).

2. Respiratory mask according to the preceding Claim, **characterized in that** the first radial expansion (13) of the outer wall (11) of the nasal cushion (9) comprises a first bevelled front face (13a) that can slide against the corresponding second bevelled front face (30a) of the abutment (30) of the mask body (21) for ensuring an easy coupling of the cushion (9) to the mask body (21), when the cushion (9) is being coupled to the mask body (21).

3. Respiratory mask according to any one of the preceding Claims, **characterized in that** the outer wall (11) of the nasal cushion (9) is facing the inner wall (12).

4. Respiratory mask according to Claim 3, **characterized in that** the outer wall (11) and the inner wall (12) of the nasal cushion (9) are parallel or quasi-parallel to each other.

5. Respiratory mask according to any one of the preceding Claims, **characterized in that** the outer wall (11) and the inner wall (12) of the nasal cushion (9) are formed at the end of the peripheral border (10).

6. Respiratory mask according to any one of the preceding Claims, **characterized in that** the outer wall (11) and the inner wall (12) of the nasal cushion (9) are formed at the end of the peripheral border (10) over the entire periphery of said peripheral border (10).

7. Respiratory mask according to any one of the preceding Claims, **characterized in that** the hollow body (1), the pair of nasal prongs (4), the peripheral border (10), the outer wall (11) and the inner wall (12) of the nasal cushion (9) are formed in one piece.

8. Respiratory mask according to Claim 7, **characterized in that** the hollow body (1), the pair of nasal prongs (4), the peripheral border (10), the outer wall (11) and the inner wall (12) of the nasal cushion (9) are moulded in one piece.

9. Respiratory mask according to any one of the preceding Claims, **characterized in that** each nasal prong (4) of the nasal cushion (9) comprises an internal gas passage (7) in fluid communication with the internal chamber (2) of the hollow body (11), and further comprises an outlet orifice (6) fluidly communicating with the internal gas passage (7).

10. Respiratory mask according to any one of the preceding Claims, **characterized in that** each nasal prong (4) of the nasal cushion (9) comprises a flexible bellow portion (5) allowing each prong (4) to pivot so as to be orientated with respect to the hollow body (1), preferably said flexible bellow portions (5) are located at the proximal end of the nasal prongs (3).

11. Respiratory mask according to any one of the preceding Claims, **characterized in that** each nasal prong (4) of the nasal cushion (9) comprises an enlarged head (3) comprising the outlet orifice (6).

12. Respiratory mask according to any one of the preceding Claims, **characterized in that** said enlarged heads (3) of the nasal cushion (9) have a generally tronconical or quasi-tronconical shape.

13. Respiratory mask according to any one of the preceding Claims, **characterized in that** the hollow body (1) and the prongs (4) of the nasal cushion (9) are made of a soft deformable material.

14. Respiratory mask (20) according to any one of the preceding Claims, **characterized in that** it further comprises a headgear (26).

15. Respiratory mask according to Claim 13, **characterized in that** the hollow body (1) and the prongs (4) of the nasal cushion (9) are made of silicone.

## Patentansprüche

1. Atemmaske (20), die einen Maskenkörper (21) umfasst, der an einem Nasenkissen (9) befestigt ist, wobei das Nasenkissen (9) einen Hohlkörper (1) mit einer Innenkammer (2) umfasst, und ein Paar Spitzen (4), die jeweils mit dem Hohlkörper (1) verbunden und mit ihm integral sind, wobei der Hohlkörper (1) ferner einen Umfangsrand (10) umfasst, der eine Verbindungsstruktur (11, 13; 12, 14) umfasst, um den Hohlkörper (1) an dem Maskenkörper (20) zu befestigen oder zu koppeln, wobei die Verbindungsstruktur (11, 13; 12, 14) eine Außenwand (11) und eine Innenwand (12), die voneinander durch eine Zwischenbeabstandung (15) getrennt sind, umfasst, **dadurch gekennzeichnet, dass**:
- die Außenwand (11) des Nasenkissens (9) eine erste radiale Erweiterung (13) umfasst, die in die Zwischenbeabstandung (15) zu der Innenwand (12) vorsteht, und wobei die Innenwand (12) eine zweite radiale Erweiterung (14) umfasst, die in die Innenkammer (2) des Hohlkörpers (1) vorsteht,
- die Außenwand (11) und die erste radiale Erweiterung (13) des Nasenkissens (9) eine hakenähnliche Struktur bilden, wobei die erste radiale Erweiterung (13) eine erste abgeschrägte Vorderseite (13a) umfasst,
- die zweite radiale Erweiterung (14) des Nasenkissens (9) einen Höcker auf der inneren Oberfläche (14a) der Innenwand (12) bildet,
- und der Maskenkörper (21) Folgendes umfasst:
-- eine Anschlagstruktur (30), die eine zweite abgeschrägte Vorderseite (30a) umfasst und mit der ersten radialen Erweiterung (13) des Kissens (9) zusammenwirkt, wenn das Kissen in dem Maskenkörper (21) angebracht wird, und
-- eine Aufnahme (28), die einen Wandabschnitt (29) hat, der mit der Höckerstruktur auf der inneren Oberfläche (14a) der Innenwand (12) der zweiten radialen Erweiterung (14) des Kissens (9) zusammenwirkt, wenn das Kissen (9) in dem Maskenkörper (21) angebracht wird,
wodurch das Nasenkissen (9) fest auf dem Maskenkörper (21) dank der Kombination von Aktionen der hakenähnlichen Struktur (13) der Außenwand (11) des Kissens (9), die mit der Anschlagstruktur (30) des Maskenkörpers (21) zusammenwirkt, und des vorstehenden Höckers, der zweiten radialen Erweiterung (14) des Kissens (9), die in einer nicht gleitenden Art mit der Oberfläche des Wandabschnitts (29) des Maskenkörpers (21) in Berührung kommt, während das Kissen (9) mit dem Maskenkörper (21) gekoppelt wird, gehalten wird,
wobei der Höcker ferner als eine Dichtung wirkt, die eine Gasabdichtung zwischen der zweiten radialen Erweiterung (14) des Kissens (9) und dem Wandabschnitt (29) des Maskenkörpers (21) sicherstellt.

2. Atemmaske nach dem vorhergehenden Anspruch, **dadurch gekennzeichnet, dass** die erste radiale Erweiterung (13) der Außenwand (11) des Nasenkissens (9) eine erste abgeschrägte Vorderseite (13a) umfasst, die an der entsprechenden zweiten abgeschrägten Vorderseite (30a) des Anschlags (30) des Maskenkörpers (21) gleiten kann, um ein leichtes Koppeln des Kissens (9) an dem Maskenkörper (21) sicherzustellen, wenn das Kissen (9) mit dem Maskenkörper (21) gekoppelt wird.

3. Atemmaske nach einem der vorhergehenden Ansprüche, **dadurch gekennzeichnet, dass** die Außenwand (11) des Nasenkissens (9) zu der Innenwand (12) zeigt.

4. Atemmaske nach Anspruch 3, **dadurch gekennzeichnet, dass** die Außenwand (11) und die Innenwand (12) des Nasenkissens (9) zueinander parallel oder fast parallel sind.

5. Atemmaske nach einem der vorhergehenden Ansprüche, **dadurch gekennzeichnet, dass** die Außenwand (11) und die Innenwand (12) des Nasenkissens (9) an dem Ende des Umfangsrands (10) ausgebildet sind.

6. Atemmaske nach einem der vorhergehenden Ansprüche, **dadurch gekennzeichnet, dass** die Außenwand (11) und die Innenwand (12) des Nasenkissens (9) an dem Ende des Umfangsrands (10) über den gesamten Umfang des Umfangsrands (10) ausgebildet sind.

7. Atemmaske nach einem der vorhergehenden Ansprüche, **dadurch gekennzeichnet, dass** der Hohlkörper (1), das Paar Nasenspitzen (4), der Umfangsrand (10), die Außenwand (11) und die Innenwand (12) des Nasenkissens (9) aus einem Teil gebildet sind.

8. Atemmaske nach Anspruch 7, **dadurch gekennzeichnet, dass** der Hohlkörper (1), das Paar Nasenspitzen (4), der Umfangsrand (10), die Außenwand (11) und die Innenwand (12) des Nasenkissens (9) aus einem Stück geformt sind.

9. Atemmaske nach einem der vorhergehenden Ansprüche, **dadurch gekennzeichnet, dass** jede Nasenspitze (4) des Nasenkissens (9) eine innere Gaspassage (7) in Fluidverbindung mit der Innenkammer (2) des Hohlkörpers (11) umfasst und ferner eine Auslassöffnung (6) in Fluidverbindung mit der inneren Gaspassage (7) umfasst.

10. Atemmaske nach einem der vorhergehenden Ansprüche, **dadurch gekennzeichnet, dass** jede Nasenspitze (4) des Nasenkissens (9) einen biegsamen Balgabschnitt (5) umfasst, der es jeder Spitze (4) erlaubt, derart zu schwenken, dass sie in Bezug zu dem Hohlkörper (1) ausgerichtet ist, wobei sich die biegsamen Balgabschnitte (5) vorzugsweise an dem proximalen Ende der Nasenspitzen (3) befinden.

11. Atemmaske nach einem der vorhergehenden Ansprüche, **dadurch gekennzeichnet, dass** jede Nasenspitze (4) des Nasenkissens (9) einen vergrößerten Kopf (3), der die Auslassöffnung (6) umfasst, umfasst.

12. Atemmaske nach einem der vorhergehenden Ansprüche, **dadurch gekennzeichnet, dass** die vergrößerten Köpfe (3) des Nasenkissens (9) im Allgemeinen kegelstumpfförmige oder fast kegelstumpfförmige Form haben.

13. Atemmaske nach einem der vorhergehenden Ansprüche, **dadurch gekennzeichnet, dass** der Hohlkörper (1) und die Spitzen (4) des Nasenkissens (9) aus einem weichen verformbaren Material hergestellt sind.

14. Atemmaske (20) nach einem der vorhergehenden Ansprüche, **dadurch gekennzeichnet, dass** sie ferner ein Kopfgeschirr (26) umfasst.

15. Atemmaske nach Anspruch 13, **dadurch gekennzeichnet, dass** der Hohlkörper (1) und die Spitzen (4) des Nasenkissens (9) aus Silikon hergestellt sind.

## Revendications

1. Masque respiratoire (20) comprenant un corps de masque (21) fixé à un coussinet nasal (9), ledit coussinet nasal (9) comprenant un corps creux (1) avec une chambre interne (2), et une paire de canules nasales (4) connectées chacune au corps creux (1) et d'un seul tenant avec celui-ci, ledit corps creux (1) comprenant en outre une bordure périphérique (10) comprenant une structure de connexion (11, 13 ; 12, 14) pour fixer ou coupler le corps creux (1) au corps de masque (20), la structure de connexion (11, 13 ; 12, 14) comprenant une paroi extérieure (11) et une paroi intérieure (12) séparées l'une de l'autre par un espacement intermédiaire (15),
**caractérisé en ce que** :
- la paroi extérieure (11) du coussinet nasal (9) comprend une première expansion radiale (13) se projetant dans l'espacement intermédiaire (15) vers la paroi intérieure (12), et ladite paroi intérieure (12) comprend une deuxième expansion radiale (14) se projetant dans la chambre interne (2) du corps creux (1),
- la paroi extérieure (11) et la première expansion radiale (13) du coussinet radial (9) forment une structure analogue à un crochet, ladite première expansion radiale (13) comprenant une première face avant biseautée (13a),
- la deuxième expansion radiale (14) du coussinet radial (9) forme un bossage sur la surface intérieure (14a) de la paroi intérieure (12),
- et le corps de masque (21) comprend:
. une structure de butée (30) comprenant une deuxième face avant biseautée (30a), et coopérant avec la première expansion radiale (13) du coussinet (9), quand le coussinet est mis en place dans le corps de masque (21), et
. un logement (28) ayant une portion de paroi (29) coopérant avec la structure de bossage sur la surface intérieure (14a) de la paroi intérieure (12) de la deuxième expansion radiale (14) du coussinet (9), quand le coussinet (9) est mis en place dans le corps de masque (21),
maintenant ainsi fermement le coussinet nasal (9) sur le corps de masque (21) grâce à la combinaison d'actions de la structure analogue à un crochet (13) de la paroi extérieure (11) du coussinet (9) qui coopère avec la structure de butée (30) du corps de masque (21), et du bossage de projection de la deuxième expansion radiale (14) du coussinet (9) qui entre en contact, de manière non coulissante, avec la surface de la portion de paroi (29) du corps de masque (21), alors que le coussinet (9) est couplé au corps de masque (21),
ledit bossage servant en outre de joint assurant une étanchéité aux gaz entre la deuxième expansion radiale (14) du coussinet (9) et la portion de paroi (29) du corps de masque (21).

2. Masque respiratoire selon la revendication précédente, **caractérisé en ce que** la première expansion radiale (13) de la paroi extérieure (11) du coussinet nasal (9) comprend une première face avant biseautée (13a) qui peut coulisser contre la deuxième face avant biseautée correspondante (30a) de la butée (30) du corps de masque (21) pour garantir un couplage facile du coussinet (9) au corps de masque (21), quand le coussinet (9) est en train d'être couplé au corps de masque (21).

3. Masque respiratoire selon l'une quelconque des revendications précédentes, **caractérisé en ce que** la paroi extérieure (11) du coussinet nasal (9) fait face à la paroi intérieure (12).

4. Masque respiratoire selon la revendication 3, **caractérisé en ce que** la paroi extérieure (11) et la paroi intérieure (12) du coussinet nasal (9) sont parallèles ou quasi-parallèles l'une à l'autre.

5. Masque respiratoire selon l'une quelconque des revendications précédentes, **caractérisé en ce que** la paroi extérieure (11) et la paroi intérieure (12) du coussinet nasal (9) sont formées à l'extrémité de la bordure périphérique (10).

6. Masque respiratoire selon l'une quelconque des revendications précédentes, **caractérisé en ce que** la paroi extérieure (11) et la paroi intérieure (12) du coussinet nasal (9) sont formées à l'extrémité de la bordure périphérique (10) sur toute la périphérie de ladite bordure périphérique (10).

7. Masque respiratoire selon l'une quelconque des revendications précédentes, **caractérisé en ce que** le corps creux (1), la paire de canules nasales (4), la bordure périphérique (10), la paroi extérieure (11) et la paroi intérieure (12) du coussinet nasal (9) sont formés d'un seul tenant.

8. Masque respiratoire selon la revendication 7, **caractérisé en ce que** le corps creux (1), la paire de canules nasales (4), la bordure périphérique (10), la paroi extérieure (11) et la paroi intérieure (12) du coussinet nasal (9) sont moulés d'un seul tenant.

9. Masque respiratoire selon l'une quelconque des revendications précédentes, **caractérisé en ce que** chacune canule nasale (4) du coussinet nasal (9) comprend un passage de gaz interne (7) en communication fluidique avec la chambre interne (2) du corps creux (11), et comprend en outre un orifice de sortie (6) en communication fluidique avec le passage de gaz interne (7).

10. Masque respiratoire selon l'une quelconque des revendications précédentes, **caractérisé en ce que** chacune canule nasale (4) du coussinet nasal (9) comprend une portion de soufflet flexible (5) permettant à chaque canule (4) de pivoter de manière à être orientée par rapport au corps creux (1), de préférence lesdites portions de soufflet flexibles (5) se trouvent au niveau de l'extrémité proximale des canules nasales (3).

11. Masque respiratoire selon l'une quelconque des revendications précédentes, **caractérisé en ce que** chacune canule nasale (4) du coussinet nasal (9) comprend une tête agrandie (3) comprenant l'orifice de sortie (6).

12. Masque respiratoire selon l'une quelconque des revendications précédentes, **caractérisé en ce que** lesdites têtes agrandies (3) du coussinet nasal (9) ont une forme généralement tronconique ou quasi-tronconique.

13. Masque respiratoire selon l'une quelconque des revendications précédentes, **caractérisé en ce que** le corps creux (1) et les canules (4) du coussinet nasal (9) se composent d'un matériau déformable mou.

14. Masque respiratoire (20) selon l'une quelconque des revendications précédentes, **caractérisé en ce qu'**il comprend en outre un harnais (26).

15. Masque respiratoire selon la revendication 13, **caractérisé en ce que** le corps creux (1) et les canules (4) du coussinet nasal (9) se composent de silicone.
